# EUROPEAN PATENT SPECIFICATION

(11) **EP 3 233 055 B1**
(45) Date of publication and mention of the grant of the patent: **25.12.2024**
(21) Application number: 15816228.9
(22) Date of filing: 17.11.2015
(51) Int. Cl.: A61K 9/00, A61P 25/04, A61P 29/00, A61K 31/167

(54) **INJECTABLE FORMULATIONS OF PARACETAMOL**
INJIZIERBARE FORMULIERUNGEN AUS PARACETAMOL
FORMULATIONS INJECTABLES DE PARACÉTAMOL

(30) Priority: 20.12.2014 IN 4102MU2014
(43) Date of publication of application: 25.10.2017
(73) Proprietor: Troikaa Pharmaceuticals Ltd, Ahmedabad, Gujarat 380054 (IN)
(72) Inventor: PATEL, Ketan R., Makarba Ahmedabad (IN); PATEL, Milan R., Makarba Ahmedabad (IN); PATEL,Asheel K., Makarba Ahmedabad (IN); SHAH, Prakashchandra J., Ahmedabad Gujarat 380015 (IN)
(74) Representative: Biggi, Cristina
(86) International application number: PCT/IB2015/058876
(87) International publication number: WO 2016/097899

(56) References cited:
- WO-A1-00/07588
- WO-A2-2012/001494

## Description

### Field of the Invention

The present invention relates to low volume intravenous injections of paracetamol and method of preparation thereof.

### Background of the Invention

Paracetamol a para-aminophenol derivative provides analgesic, antipyretic and weak anti-inflammatory activity. It is widely used over-the-counter analgesic and antipyretic. It is commonly used for the relief of headaches and other minor aches and pains as well as fever. It is also used in combination with opioid analgesics for the management of severe pain such as post-surgical pain and providing palliative care in advanced cancer patients.

Paracetamol or Acetaminophen is available in a variety of dosage forms such as tablets, capsules, Liquid syrups, suspensions, suppositories etc. that can be administered through different routes of administration. The oral and parenteral routes are the most preferred routes of administration. (Jarde O., Boccard E., Parenteral versus Oral Route Increases Paracetamol Efficacy. Clinical Drug Investigation, Dec 1997, 14(6): 474-481.) The parenteral route of administration is preferred due to the issue of rapid degradation and poor absorption of drug following oral administration. Further, the parenteral route of administration is the only route available for effective management of the patient's clinical conditions when rapid absorption of drug is essential for quicker onset of action and faster relief from the symptoms.

In case of emergency or when patients are unconscious or unable to accept oral medications, the parenteral route is the preferred route. Drug absorption after parenteral delivery is rapid and, blood levels attained are more favourable than those achieved by oral dosage forms. (Aulton M. E.: Pharmaceutics, The Science of Dosage Form Design. Churchill Livingstone, Second edition, 5)

Other advantages of the parenteral formulations of paracetamol are that they can be administered before or during the surgery thereby permitting the initiation of effective analgesia in the early phase of the postoperative period. (Pasero C., The Role of Intravenous Acetaminophen in Acute Pain Management. Pain Management Nursing, Jun 2012, 13(2): 107-124.) These injections avoid first-pass hepatic exposure and metabolism via portal circulation, which reduces the incidence of hepatic injury. These injections rarely cause hepatotoxicity, and has been shown to be safe for use in patients with underlying liver conditions. (Viscusi E. R., IV Acetaminophen Improves Pain Management and Reduces Opioid Requirements in Surgical Patients: A Review of the Clinical Data and Case-based Presentations, April 2012, 38(4): 1-8.)

Despite the advantages of the parenteral formulations of paracetamol, very few options of parenteral formulations of paracetamol are available in the market. The key reasons for their non-availability are the drug's poor aqueous solubility and instability in presence of water,

Most of the formulations of Paracetamol are available in the form of very dilute aqueous solutions and are suitable for administration exclusively via intravenous infusion route. These large volume formulations are associated with the disadvantages related to volume overload especially in patients with compromised heart and kidney functions. In addition to their long duration of administration, and necessity to maintain a specific infusion rate etc the manufacturing cost of these formulations is relatively high. (Swarbrick J., Encyclopedia of Pharmaceutical Technology, Informa Healthcare USA, Third Edition, 1001-1003.)

WO2009098716 and WO2009/047634 disclose stable aqueous pharmaceutical compositions comprising paracetamol for parenteral administration, wherein the concentration of Paracetamol in the composition is 10mg/ml and therefore the said compositions are suitable only for administration via Intra-Venous (IV) infusion. WO2003033026 discloses ready to use stable injectable formulations wherein the concentration of Paracetamol is upto 40 mg/ml for administration only by IV infusion route.

WO2001008662 is related to pharmaceutical compositions of Paracetamol comprising atleast 10% w/v of Paracetamol in anhydrous PEG200. Viscosities of these compositions are of the order of 168 Cps and are therefore have limited use through parenteral route only upon dilution.

IN1746/MUM/2008 relates to injectable formulations of Paracetamol only upto 15% prepared in a solvent system of Glycofurol & water. These formulations provide only upto 150 mg of drug in 1 ml.

WO00/07588 discloses non-aqueous injectable formulations of Paracetamol using alcohol and polyethylene glycol. These injections are formulated in non-aqueous solvents and are not in the ready-to-use form. The non-aqueous formulations disclosed in the said reference are not suitable for intramuscular (IM) administration without dilution with aqueous fluid.

In US Patent 6028222, column 3 lines 47 to 53 mentions aqueous formulations of paracetamol injections with the Paracetamol concentrations in the range of 2mg/ml to 50 mg/ml in dilute solutions and 60 mg/ml to 350 mg/ml in concentrated solutions. However in column 6 of the same reference specification, the said US patent also states the following:
*"Using this solution composed of a solvent mixture constituted by 30% of propyleneglycol, by 40% of polyethylene-glycol 400 and by 30% of water (solution no 20), it is possible to dissolve about 200 mg*/*ml of paracetamol at 20° C. Choosing a concentration of 160 mg*/*ml allows one to be sure that no recristallization will occur "*

Thus by their own admission, the inventors in the said US Patent therefore teach that concentration of 160 mg/ml result in injectables in which no recrystallization occurs. The inventors of US'222 themselves admit and illustrate by way of examples that the formulations containing Paracetamol concentration upto 160 mg/ml remain stable. There is no enabling disclosure or teaching of whether concentrations as high as 350 mg/ml will yield an injectable where no recrystallization occurs. It is not obvious whether concentrations in the range 160 mg/ml to 350 mg/ml would be workable based on the teachings of US'222.

When the formulations disclosed in the specification of US'222 with high concentration of paracetamol (≥ 160 mg/ml) were prepared by us and diluted to obtain a dose of 1 gm in not more than 20 ml, (i.e. to provide the concentration of atleast 50 mg/ml), the drug crystallised out of these solutions in 3 to 5 minutes. These formulations are therefore not appropriate for slow IV bolus administration after dilution, as crystals appear within 5 minutes of dilution. The results of our studies are summarised below:

**Table 1: Results of dilution of samples of examples disclosed in column 7 of the specification of US6028222**

| **Exa mple No.** | **Concentrati on reported in US'222 @ 25 °C (mg/ml)** | **Concent rated solution taken (ml)** | **WFI added to make volume up to (ml)** | **Crystallization at 25°C observed within (minutes)** | **Crystallization at 20°C observed within (minutes)** |
|---|---|---|---|---|---|
| 3 | 230 | 4.3 | 20 | 5 | 2.5 |
| 20 | 200 | 5.0 | 20 | 3 | 1 |
| 21 | 210 | 4.7 | 20 | 4 | 2 |
| 22 | 220 | 4.5 | 20 | 3 | 1.5 |
| 17 | 200 | Crystallisation observed immediately in the concentrated solution. | | | |
| 19 | 190 | Complete solubilisation was not achieved | | | |

It is clear from the above that these formulations are unsuitable for administration through the injectable route. Our studies unequivocally establish and confirm the admission of the inventors of US'222 that the concentrated formulations of the said US patent are indeed unstable due to re-crystallisation of the drug.

WO2012001494 discloses high concentration aqueous formulations of paracetamol which deliver 500mg of the drug in a volume of injection as low as 2 to 3 ml maintaining the viscosity in the range of 7 to 28 CPs when measured by Ostwald viscometer at 25 °C. These formulations suffer from side effects such as phlebitis rendering them unsuitable for IV bolus route and therefore, without dilution, intra-muscular route is the only route of administration available for these formulations. We carried out detailed pre-clinical toxicity studies of the formulations disclosed in WO2012001494.

Repeated dose intravenous toxicity studies were carried out in 48 Swiss Albino Mice and 24 New Zealand White Rabbits for 14 consecutive days. The test solution of the paracetamol injections disclosed in WO2012001494 were prepared and administered through intravenous route (tail vein) without any dilution at the dose levels of 5 (Low), 15 (Mid) and 25 (high) µL/20 gm body wt of the Swiss Albino Mice. These formulations caused severe adverse effects at the site of injection and animals from low and mid dose Paracetamol treated groups were less affected when compared to high dose Paracetamol treated groups. Further, when these formulations were administered through marginal ear veins of New Zealand White Rabbits at the dose level of 500, 1500 and 3000 µL/2 kg body weight/day, they caused adverse effects at the site of injection. The findings of our studies in mice and rabbit suggest that the formulation of paracetamol disclosed in WO2012001494 when administered without any dilution through IV bolus route, are not tolerated even at the lowest dose. They cause severe phlebitis and are not suitable for intravenous bolus administration in humans.

When these formulations are diluted to achieve 1 gm of drug in 20 ml of formulation, precipitation of the drug sets in thereby making it unsuitable for administion through slow IV bolus route.

Some of the paracetamol injections disclosed in the prior art are non-aqueous and therefore not in ready-to use form. Further a severe limitation of prior art formulations is viscosities > 45 Cps which may cause tissue damage and pain at the site of injections. Through the present specification, the viscosity unit 1 Cps corresponds to 0.001 Pa s.

The aqueous injectable formulations of Paracetamol available till date either contain very low concentration of the drug which are administered only via the intravenous infusion route, or they are formulations containing fairly high concentrations of the drug but suffer from severe limitations such as:
a) Without dilution are capable of being administered only through intra-muscular route
b) On dilution with any of the available large volume of compatible parenterals, are suitable for administration only by the intravenous infusion route.

It is not possible to dilute the concentrated formulations known in the art to a volume of about 20 ml which can enable their administration through slow intravenous bolus route wherein the entire dose of the drug (e.g 1 gm in 20 ml ) is injected within 5 minutes, preferably in 2 minutes to a patient. This limitation results from the appearance of crystals of the drug within minutes of the dilution of the prior art formulations.

There is an unmet need to provide aqueous injections of Paracetamol which are not only stable and suitable for administration through IM & IV infusion route but are also suitable for administration through slow intravenous bolus injections route but exhibiting minimal or no effects. In addition to providing stable high concentration solutions, these ready to use formulations must have viscosities of < 0.035 Pa s (< 35 Cps), preferably < 0.025 Pa s (< 25 Cps) at room temperature.

There exists a need to provide stable formulations which, enable administration of the therapeutic dose of 1 gm in a volume not more than 20 ml with viscosities < 0.035 Pa·s (<35 Cps), preferably < 0.025 Pa·s (< 25 Cps) at room temperature, thereby rendering them suitable for administration through slow intravenous bolus route upon dilution with aqueous fluids over and above the Intramuscular, Intravenous infusion routes.

### Objects of the Invention:

The main object of the present invention is to provide high concentration injectable formulations of paracetamol which when diluted with aqueous fluids are capable of providing a dose of 1 gm of the drug in not more than 20 ml without any crystallisation of drug for a duration of atleast 5 minutes after dilution.

It is yet another object of the present invention to provide high concentration stable aqueous injections of paracetamol which, when diluted with aqueous fluids to deliver 1 gm of drug in not more than 20 ml of the formulation, are suitable for being administered through slow intravenous bolus route.

Another object of the present invention is to provide high concentration stable aqueous injections of paracetamol which can be administered through intra-muscular route in their undiluted form and are also suitable for slow IV bolus administration with minimised side effects such as phlebitis, pain etc.

### Detailed Description of the Invention:

The present invention provides stable high concentration aqueous injections of paracetamol which when diluted with aqueous fluids to not more than 20 ml, remain stable and are suitable for administration through slow intravenous route.

An injectable formulation of the present invention comprises
250 mg/ml paracetamol in a solvent system comprising
(a) less than or equal to 40% w/v glycofurol,
(b) less than or equal to 30% w/v lower chain alcohol selected from the group comprising ethyl alcohol, iso-propyl alcohol, or mixture thereof,
(c) 2% to 4% w/v stabilizer selected from Plasdone C 17, Plasdone C 30 or its combination thereof,
(d) optionally another solvent selected from N-methyl pyrrolidone, cyclodextrin, dimethylacetamide, transcutol, or mixtures thereof, and
(e) water to make up the volume of the formulation.

The another solvent is preferably selected from N-methyl pyrrolidone, dimethylacetamide, transcutol, cyclodextrin, or mixtures thereof.

The amount of glycofurol in the formulations is less than or equal to 40 % w/v, preferably less than or equal to 35 % w/v of the formulation. The amount of lower chain alcohol is less than or equal to 30 % w/v, preferably less than or equal to 25 % w/v of the formulation. The amount of another solvent in the formulations is less than or equal to 55 % v/v of the formulation.

The stabiliser is selected from grades of polymeric compounds such as plasdone for example plasdone C 17, plasdone C 30 or its combination thereof. The amount of stabiliser is 2 % w/v to 4 % w/v of the formulation.

In one embodiment the injectable containing 250 mg paracetamol in 1 ml. In another embodiment, the dose of 500 mg of drug can be delivered in a volume of 2 ml. In another embodiment, the dose of 1 gm can be delivered in a volume of 4 ml. These embodiments are suitable for administration through the Intramuscular route.

In one of the embodiments, these formulations when diluted using low volume of aqueous fluids, can deliver the therapeutic dose of 1 gm of Paracetamol in 20 ml of the formulation without any recrystallization for atleast upto 5 minutes after dilution, in another embodiment, such formulations do not exhibit recrystallization in atleast 8 to 20 minutes after dilution.

The aqueous fluids for dilution are selected from water for injection or any other medicated/ non-medicated aqueous fluids suitable for slow IV bolus administration. These formulations also provide the option of dilution with large volume of aqueous fluids in order to deliver the drug through intravenous infusion route.

These formulations result in complete solubilisation of Paracetamol. The formulations containing 1 gm of drug when diluted with aqueous fluids upto 20 ml, do not exhibit and recrystallization atleast up to 5 minutes after dilution, more preferably up to a period of atleast 8 minutes after dilution.

Injectable formulations comprising 250 mg/ml of paracetamol, the solvent system of the present invention with a stabiliser in a specified range provides injectable formulations which on dilution with aqueous fluids up to 20 ml of formulation, are suitable for slow IV bolus route of administration with significantly minimised side effects such as phlebitis.

The formulations of the present invention are stable throughout the shelf life of the formulations. In the present invention, water is used in a quantity sufficient to make up the final volume of the formulation. Water to make up the final volume in these formulations is less than or equal to 50 % w/v, preferably less than or equal to 45 % w/v of the formulations.

The said formulations of the present invention can further be diluted with the help of water for injection or conventional aqueous IV fluids such as normal saline, dextrose solution or any other parenteral carriers known in the art such that a dose of 1 gm of Paracetamol is delivered in 20 ml volume of the final formulation.

In another embodiment of the present formulations, suitable auxiliary ingredients such as antioxidant(s), pH modifier(s), buffer(s), chelating agent(s), or mixtures thereof may be used. The antioxidant(s) of the formulation can be selected from monothioglycerol, sodium metabisulphite. The pH modifier(s) the formulation can be selected from sodium hydroxide, hydrochloric acid. The buffer(s) of the formulation can be selected from dibasic sodium phosphate, monobasic sodium phosphate.

The said auxiliary ingredients are used in the pharmaceutically acceptable proportions known in the art.

The viscosity of the present formulations in their undiluted form is in the range of 0.005 Pa·s to 0.035 Pa·s (5 to 35 CPs) when measured by Ostwald viscometer at 25°C. The viscosity of the preferred embodiments of the present formulations in their undiluted form ranges from 0.008 Pa·s to 0.032 Pa·s (8 to 32 CPs) at 25°C. This feature of the formulations of the present invention not only provides the advantage of less or no pain and no tissue damage at the site of injection when administered in undiluted form intra-muscularly but also leads to less or no pain when administered upon dilution upto 20ml via slow IV bolus route over a period of 2 to 5 minutes.

The applicant conducted pre-clinical (animal) toxicity studies to establish the above mentioned advantageous features of the formulation of the present invention. The said studies and results thereof are summarised as under.

### Sub-acute toxicity study of Undiluted Paracetamol injection (1000 mg/4 ml) in rats and mice:

The study was aimed to assess toxicity of paracetamol injection 1000 mg/4 ml (250 mg/ml) in rats and mice after repeated administration for 28 days. The animals were selected, conditioned, and exposed administered the test compound through Intra venous undiluted bolus injection at three dosage levels (15, 25, 35 mg/kg) and observed for 28 days.

All animals were observed for abnormal clinical signs and behavioural changes; morbidity and mortality; changes in body weight and food consumption throughout the study period. At the end of study (i.e. 28 days), the animals were also evaluated for haematological and biochemical estimations and histopathology of different organs for determination of toxicity.

The results showed that there were no mortalities observed among the animals treated due to test compound. There was no significant treatment effect on food intake, body weight gain and clinical signs, behavioural activity etc. Further, there were no significant changes found in haematological and biochemical parameters of mice and rats treated with all three dose levels of paracetamol as compared to control vehicle group.

The result concluded that surprisingly no toxicity was observed in rats and mice when undiluted Paracetamol formulation (1000 mg/4 ml) was injected by intravenous bolus route of administration. This is in contrast with the results of the study conducted for the formulations in WO2012001494 which were not tolerated through IV bolus route even at low dose.

### Repeated dose toxicity study of Paracetamol injection (1000 mg/4 ml) in rabbits

The objective of this study was to assess the local and systemic toxicity in rabbits from 14 days repeated administration of Paracetamol Injection (1000 mg/4 ml), prepared as per the present invention with a special emphasis on identifying target organs toxicities.

In this study, animals were randomly assigned to four treatment groups. Each group comprised of two female rabbits. Two groups (01 & 02) were given vehicle (Placebo) for Paracetamol Injection (1000 mg/4 ml) diluted upto 20 ml with water for injection. Other two groups (03 & 04) were given Paracetamol Injection (1000 mg/4 ml) diluted upto 20 ml with water for injection. 01 & 03 groups were treated with their respective treatments as once a day dose at volume of 2.52 ml/ 2 Kg Rabbit. 02 & 04 groups were treated with their respective treatments in divided doses administered four times a day (0.63 ml/2 Kg each time). The diluted injections were administered as slow intravenous infusion at 65 mg/kg dose level within 2 to 5 minutes.

All the animals were observed once a day for signs of toxicity throughout the experimental period and twice daily for mortality and morbidity. Body weights were recorded on Day 1, 8, and 14. Feed consumption was calculated on weekly basis. At the end of experiment (Day 14), all surviving animals were sacrificed and gross pathology data was recorded.

At the end of study (i.e. 14 days), the animals were evaluated for gross pathology and histopathology of different organs including site of injection for determination of toxicity. The result showed no local or systemic toxicity with Paracetamol injection 1000 mg/4 ml when administered as intravenous infusion in diluted form with water for injection for 14 days in rabbits.

The results of the preclinical studies conducted by the applicant confirmed that the formulations of the present invention are capable of being administered through IV bolus route of administration in their undiluted form as well as through slow IV bolus route of administration over 2 to 5 minutes when diluted with water for injection to a final volume upto 20 ml.

Non-limiting examples of the formulations of the present invention are provided herein below

### Example 1

| **Ingredients** | **Amount (mg/ml)** | **Percentage %** |
|---|---|---|
| Paracetamol | 250 | 25% w/v |
| Glycofurol | 353 | 35.3% w/v |
| Ethyl alcohol | 240 | 24% w/v |
| Plasdone C 17 | 20 | 2% w/v |
| Water for Injection | Qs to 1 ml | Qs to 100% |

The viscosity of the above formulation (undiluted) was 0.018 Pa s (18 CPs) when measured by Ostwald viscometer at 25°C.

When 4 ml (volume equivalent to 1 gm) of the above formulation was diluted with water for injections upto a final volume of 20 ml, no recrystallization of drug was observed at 25°C up to duration of 8 minutes after dilution and at 20°C up to duration of 5 minutes after dilution.

### Example 2:

| **Ingredients** | **Amount (mg/ml)** | **Percentage %** |
|---|---|---|
| Paracetamol | 250 | 25% w/v |
| Glycofurol | 300 | 30.0% w/v |
| Ethyl alcohol | 300 | 30% w/v |
| Plasdone C 30 | 25 | 2.5% w/v |
| Water for Injection | Qs to 1 ml | Qs to 100% |

The viscosity of the above formulation (undiluted) was 0.015 Pa s (15 CPs) when measured by Ostwald viscometer at 25°C.

When 4 ml (volume equivalent to 1 gm) of the above formulation was diluted with water for injections upto a final volume of 20 ml, no recrystallization of drug was observed at 25°C up to duration of 8 minutes after dilution and at 20°C up to duration of 5 minutes after dilution.

### Example 3:

| **Ingredients** | **Amount (mg/ml)** | **Percentage %** |
|---|---|---|
| Paracetamol | 250 | 25% w/v |
| Glycofurol | 353 | 35.3% w/v |
| Ethyl alcohol | 240 | 24% w/v |
| Plasdone C 17 | 30 | 3% w/v |
| Water for Injection | Qs to 1 ml | Qs to 100% |

The viscosity of the above formulation (undiluted) was 0.01946 Pa·s (19.46 CPs) when measured by Ostwald viscometer at 25°C.

When 4 ml (volume equivalent to 1 gm) of the above formulation was diluted with water for injections upto a final volume of 20 ml, no recrystallization of drug was observed at 25°C up to duration of 9 minutes after dilution and at 20°C up to duration of 8 minutes after dilution.

### Example 4:

| **Ingredients** | **Amount (mg/ml)** | **Percentage %** |
|---|---|---|
| Paracetamol | 250 | 25% w/v |
| Glycofurol | 353 | 35.3% w/v |
| Ethyl alcohol | 240 | 24% w/v |
| Plasdone C 17 | 40 | 4% w/v |
| Water for Injection | Qs to 1 ml | Qs to 100% |

The viscosity of the above formulation (undiluted) was 0.02094 Pa·s (20.94 CPs) when measured by Ostwald viscometer at 25°C.

When 4 ml (volume equivalent to 1 gm) of the above formulation was diluted with water for injections upto a final volume of 20 ml, no recrystallization of drug was observed at 25°C up to duration of 11 minutes after dilution and at 20°C up to duration of 8 minutes after dilution

### Example 5:

| **Ingredients** | **Amount (mg/ml)** | **Percentage %** |
|---|---|---|
| Paracetamol | 250 | 25 %w/v |
| Glycofurol | 353 | 35.3 %w/v |
| Ethyl alcohol | 224 | 22.4 %w/v |
| Plasdone C 17 | 20 | 2 %w/v |
| N-Methyl Pyrrolidone | 20.6 | 2.06 %w/v |
| Water for Injection | Qs to 1 ml | Qs to 100% |

The viscosity of the above formulation (undiluted) was 0.01673 Pa·s (16.73 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Example 6:

| **Ingredients** | **Amount (mg/ml)** | **Percentage %** |
|---|---|---|
| Paracetamol | 250 | 25 %w/v |
| Glycofurol | 353 | 35.3 %w/v |
| Ethyl alcohol | 160 | 16 %w/v |
| Plasdone C 17 | 20 | 2 %w/v |
| N-Methyl Pyrrolidone | 103 | 10.3 %w/v |
| Water for Injection | Qs to 1 ml | Qs to 100% |

The viscosity of the above formulation (undiluted) was 0.1646 Pa·s (16.46 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Example 7:

| **Ingredients** | **Amount (mg/ml)** | **Percentage %** |
|---|---|---|
| Paracetamol | 250 | 25 %w/v |
| Glycofurol | 353 | 35.3 %w/v |
| Ethyl alcohol | 80 | 8 %w/v |
| Plasdone C 17 | 20 | 2 %w/v |
| N-Methyl Pyrrolidone | 206 | 20.6 %w/v |
| Water for Injection | Qs to 1 ml | Qs to 100% |

The viscosity of the above formulation (undiluted) was 0.02294 Pa·s (22.94 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Example 8:

| **Ingredients** | **Amount (mg/ml)** | **Percentage %** |
|---|---|---|
| Paracetamol | 250 | 25 %w/v |
| Glycofurol | 270 | 27 %w/v |
| Ethyl alcohol | 240 | 24 %w/v |
| Plasdone C 17 | 20 | 2 %w/v |
| N-Methyl Pyrrolidone | 61.8 | 6.18 %w/v |
| Water for Injection | Qs to 1 ml | Qs to 100% |

The viscosity of the above formulation (undiluted) was 0.0151 Pa·s (15.1 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Example 9:

| **Ingredients** | **Amount (mg/ml)** | **Percentage %** |
|---|---|---|
| Paracetamol | 250 | 25 %w/v |
| Glycofurol | 244.9 | 24.49 %w/v |
| Ethyl alcohol | 240 | 24 %w/v |
| Plasdone C 17 | 20 | 2 %w/v |
| N-Methyl Pyrrolidone | 103 | 10.3 %w/v |
| Water for Injection | Qs to 1 ml | Qs to 100% |

The viscosity of the above formulation (undiluted) was 0.01176 Pa·s (11.76 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Comparative example 10:

| **Ingredients** | **Amount (mg/ml)** | **Percentage %** |
|---|---|---|
| Paracetamol | 250 | 25 %w/v |
| Glycofurol | 270 | 27 %w/v |
| Ethyl alcohol | 240 | 24 %w/v |
| N-Methyl Pyrrolidone | 61.8 | 6.18 %w/v |
| Water for Injection | Qs to 1 ml | Qs to 100% |

The viscosity of the above formulation (undiluted) was 0.0132 Pa·s (13.2 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 8 minutes after dilution.

### Example 11:

| **Ingredients** | **Amount (mg/ml)** | **Percentage %** |
|---|---|---|
| Paracetamol | 250 | 25 %w/v |
| Glycofurol | 353 | 35.3 %w/v |
| Plasdone C 17 | 20 | 2 %w/v |
| N-Methyl Pyrrolidone | 309 | 30.9 %w/v |
| Water for Injection | Qs to 1 ml | Qs to 100% |

The viscosity of the above formulation (undiluted) was 0.03022 Pa·s (30.22 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Example 12:

| **Ingredients** | **Amount (mg/ml)** | **Percentage %** |
|---|---|---|
| Paracetamol | 250 | 25 %w/v |
| Ethyl alcohol | 240 | 24 %w/v |
| Plasdone C 17 | 20 | 2 %w/v |
| N-Methyl Pyrrolidone | 257.5 | 25.75 %w/v |
| Water for Injection | Qs to 1 ml | Qs to 100% |

The viscosity of the above formulation (undiluted) was 0.00841 Pa·s (8.41 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Comparative example 13:

| **Ingredients** | **Amount (mg/ml)** | **Percentage %** |
|---|---|---|
| Paracetamol | 250 | 25 %w/v |
| Ethyl alcohol | 160 | 16 %w/v |
| N-Methyl Pyrrolidone | 439.6 | 43.96 %w/v |
| Water for Injection | Qs to 1 ml | Qs to 100% |

The viscosity of the above formulation (undiluted) was 0.00929 Pa·s (9.29 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Example 14:

| **Ingredients** | **Amount (mg/ml)** | **Percentage %** |
|---|---|---|
| Paracetamol | 250 | 25 %w/v |
| Ethyl alcohol | 80 | 8 %w/v |
| Plasdone C 17 | 20 | 2 %w/v |
| N-Methyl Pyrrolidone | 412 | 41.2 %w/v |
| Water for Injection | Qs to 1 ml | Qs to 100% |

The viscosity of the above formulation (undiluted) was 0.01112 Pa·s (11.12 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Comparative example 15:

| **Ingredients** | **Amount (mg/ml)** | **Percentage %** |
|---|---|---|
| Paracetamol | 250 | 25 %w/v |
| Ethyl alcohol | 80 | 8 %w/v |
| N-Methyl Pyrrolidone | 542.6 | 54.26 %w/v |
| Water for Injection | Qs to 1 ml | Qs to 100% |

The viscosity of the above formulation (undiluted) was 0.01061 Pa·s (10.61 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Comparative example 16:

| **Ingredients** | **Amount (mg/ml)** | **Percentage %** |
|---|---|---|
| Paracetamol | 250 | 25 %w/v |
| Plasdone C 17 | 20 | 2 %w/v |
| N-Methyl Pyrrolidone | 386.2 | 3 8.62 %w/v |
| Water for Injection | Qs to 1 ml | Qs to 100% |

The viscosity of the above formulation (undiluted) was 0.01024 Pa·s (10.24 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Example 17:

| **Ingredients** | **Amount (mg/ml)** | **Percentage %** |
|---|---|---|
| Paracetamol | 250 | 25 %w/v |
| Glycofurol | 150 | 15 %w/v |
| Ethyl alcohol | 250 | 25 %w/v |
| Plasdone C 17 | 20 | 2 %w/v |
| N-Methyl Pyrrolidone | 248 | 24.8 %w/v |
| Water for Injection | Qs to 1 ml | Qs to 100% |

The viscosity of the above formulation (undiluted) was 0.0126 Pa·s (12.6 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Example 18:

| **Ingredients** | **Amount (mg/ml)** | **Percentage %** |
|---|---|---|
| Paracetamol | 250 | 25 %w/v |
| Glycofurol | 200 | 20 %w/v |
| Ethyl alcohol | 300 | 30 %w/v |
| Plasdone C 17 | 20 | 2 %w/v |
| N-Methyl Pyrrolidone | 156 | 15.6 %w/v |
| Water for Injection | Qs to 1 ml | Qs to 100% |

The viscosity of the above formulation (undiluted) was 0.0145 Pa·s (14.5 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Example 19:

| **Ingredients** | **Amount (mg/ml)** | **Percentage %** |
|---|---|---|
| Paracetamol | 250 | 25 %w/v |
| Glycofurol | 353 | 35.3 %w/v |
| Ethyl alcohol | 200 | 20 %w/v |
| Plasdone C 17 | 50 | 5 %w/v |
| N-Methyl Pyrrolidone | 32.5 | 3.25 %w/v |
| Water for Injection | Qs to 1 ml | Qs to 100% |

The viscosity of the above formulation (undiluted) was 0.0211 Pa·s (21.1 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

### Example 20:

| **Ingredients** | **Amount (mg/ml)** | **Percentage %** |
|---|---|---|
| Paracetamol | 250 | 25 %w/v |
| Glycofurol | 400 | 40 %w/v |
| Ethyl alcohol | 90 | 9 %w/v |
| Plasdone C 17 | 20 | 2 %w/v |
| N-Methyl Pyrrolidone | 164 | 16.4 %w/v |
| Water for Injection | Qs to 1 ml | Qs to 100% |

The viscosity of the above formulation (undiluted) was 0.0202 Pa·s (20.2 CPs) when measured by Ostwald viscometer at 25°C.

When 4 ml (volume equivalent to 1 gm) of the above formulation was diluted with water for injections up to a final volume of 20 ml, no recrystallization of drug was observed at 25°C up to duration of 20 minutes after dilution.

The present invention has surprisingly found that Injectable formulations comprising 250 mg/ml paracetamol, a solvent system comprising Glycofurol, Lower chain alcohol, a stabiliser, water which when diluted with aqueous fluids to not more than 20 ml remain stable. In another embodiment of the present formulation, another solvent component may be used. These formulations are also suitable for slow IV bolus administration with minimised side effects such as phlebitis.

## Claims

1. An injectable formulation of paracetamol comprising:
250 mg/ml paracetamol in a solvent system comprising
(a) less than or equal to 40% w/v glycofurol,
(b) less than or equal to 30% w/v lower chain alcohol selected from the group comprising ethyl alcohol, iso-propyl alcohol, or mixture thereof,
(c) 2% to 4% w/v stabilizer selected from Plasdone C 17, Plasdone C 30 or its combination thereof,
(d) optionally another solvent selected from N-methyl pyrrolidone, cyclodextrin, dimethylacetamide, transcutol, or mixtures thereof; and
(e) water to make up the volume of the formulation.

2. The injectable formulation of claim 1, wherein the viscosity of the formulation ranges from 0.005 Pa·s to 0.035 Pa·s (5 to 35 CPs) at 25°C, preferably 0.008 Pa·s to 0.032 Pa·s (8 to 32 CPs).

3. The injectable formulation of claims 1-2, wherein the amount of water is less than or equal to 50 % v/v, of the formulation.

4. The injectable formulation of claims 1-3, wherein the formulation further comprises auxiliary ingredients such as antioxidant(s), pH modifier(s), buffer(s), chelating agent(s), or any mixtures thereof.

5. The injectable formulation of claims 1-4, wherein the antioxidant(s) of the formulation are selected from a group comprising monothioglycerol, sodium metabisulphite.

6. The injectable formulation of claims 1-5, wherein the pH modifier(s) of the formulation are selected from a group comprising sodium hydroxide, hydrochloric acid.

7. The injectable formulation of claims 1-6, wherein the buffer(s) of the formulation are selected from a group comprising dibasic sodium phosphate, monobasic sodium phosphate.

8. The injectable formulation of claims 1-7, wherein the formulation when diluted with the help of water for injection or conventional aqueous IV fluids such as normal saline, dextrose solution, or any other parenteral carriers, such that a dose of 1 gm of paracetamol is delivered in 20 ml volume of the final formulation, no crystals appear for a period of atleast 5 to 8 minutes.

9. The injectable formulation of claims 1-8, wherein the formulations are capable of administration via intra-muscular, intra-venous bolus, intravenous infusion as well as slow intra-venous bolus injection.

10. The injectable formulation of claims 1-9, wherein the slow intravenous bolus injection is stable from 2 to 10 minutes after dilution of the formulation.

## Patentansprüche

1. Injizierbare Formulierung aus Paracetamol, umfassend: 250 mg/ml Paracetamol in einem Lösungsmittelsystem, umfassend:
(a) weniger als oder gleich 40 % G/V Glycofurol,
(b) weniger als oder gleich 30 % G/V niederkettiger Alkohol, ausgewählt aus der Gruppe, umfassend Ethylalkohol, Isopropylalkohol oder eine Mischung davon,
(c) 2 % bis 4 % G/V Stabilisator, ausgewählt aus Plasdone C 17, Plasdone C 30 oder deren Kombination davon,
(d) optional ein anderes Lösungsmittel, ausgewählt aus N-Methylpyrrolidon, Cyclodextrin, Dimethylacetamid, Transcutol oder Mischungen davon; und
(e) Wasser, um das Volumen der Formulierung auszugleichen.

2. Injizierbare Formulierung nach Anspruch 1, wobei die Viskosität der Formulierung im Bereich von 0,005 Pa·s bis 0,035 Pa-s (5 bis 35 CPs) bei 25 °C, vorzugsweise 0,008 Pa-s bis 0,032 Pa-s (8 bis 32 CPs) liegt.

3. Injizierbare Formulierung nach den Ansprüchen 1-2, wobei die Wassermenge weniger als oder gleich 50 % v/v der Formulierung beträgt.

4. Injizierbare Formulierung nach den Ansprüchen 1-3, wobei die Formulierung ferner Hilfsstoffe wie Antioxidationsmittel, pH-Modifikator(en), Puffer, Chelatbildner oder beliebige Mischungen davon umfasst.

5. Injizierbare Formulierung nach den Ansprüchen 1-4, wobei das/die Antioxidationsmittel der Formulierung aus einer Gruppe ausgewählt sind, die Monothioglycerin, Natriummetabisulfit umfasst.

6. Injizierbare Formulierung nach den Ansprüchen 1-5, wobei der/die pH-Modifikator(en) der Formulierung aus einer Gruppe ausgewählt sind, die Natriumhydroxid, Salzsäure umfasst.

7. Injizierbare Formulierung nach den Ansprüchen 1-6, wobei der/die Puffer der Formulierung aus einer Gruppe ausgewählt sind, die zweibasisches Natriumphosphat, einbasisches Natriumphosphat umfasst.

8. Injizierbare Formulierung nach den Ansprüchen 1-7, wobei in der Formulierung, wenn sie mit Hilfe von Wasser für Injektionszwecke oder herkömmlichen wässrigen Infusionsflüssigkeiten wie normaler Kochsalzlösung, Dextroselösung oder einem beliebigen anderen parenteralen Träger verdünnt wird, so dass eine Dosis von 1 gm Paracetamol in einem Volumen von 20 ml der endgültigen Formulierung verabreicht wird, für einen Zeitraum von mindestens 5 bis 8 Minuten keine Kristalle erscheinen.

9. Injizierbare Formulierung nach den Ansprüchen 1-8, wobei die Formulierungen in der Lage sind, über intramuskuläre, intravenöse Bolusinjektion, intravenöse Infusion sowie langsame intravenöse Bolusinjektion verabreicht zu werden.

10. Injizierbare Formulierung nach den Ansprüchen 1-9, wobei die langsame intravenöse Bolusinjektion 2 bis 10 Minuten nach Verdünnung der Formulierung stabil ist.

## Revendications

1. Formulation injectable de paracétamol, comprenant :
250 mg/ml de paracétamol dans un système solvant, comprenant :
(a) une quantité inférieure ou égale à 40 % p/v de glycofurol,
(b) une quantité inférieure ou égale à 30 % p/v d'alcool à chaîne inférieure choisi dans le groupe comprenant l'alcool éthylique, l'alcool isopropylique ou un mélange de ceux-ci,
(c) 2 à 4 % p/v de stabilisateur choisi parmi le Plasdone C 17, le Plasdone C 30 ou leur combinaison,
(d) éventuellement, un autre solvant choisi parmi le n-méthyl pyrrolidone, la cyclodextrine, le diméthylacétamide, le Transcutol ou leurs mélanges ; et
(e) de l'eau pour compléter le volume de la formulation.

2. Formulation injectable selon la revendication 1, dans laquelle la viscosité de la formulation est comprise entre 0,005 et 0,035 Pa.s (5 à 35 cP) à 25 °C, de préférence entre 0,008 et 0,032 Pa.s (8 à 32 cP).

3. Formulation injectable selon les revendications 1-2, dans laquelle la quantité d'eau est inférieure ou égale à 50 % v/v de la formulation.

4. Formulation injectable selon les revendications 1-3, dans laquelle la formulation comprend de plus des ingrédients auxiliaires tels qu'(e) un/des antioxydant(s), un/des modificateur(s) de pH, un/des tampon(s), un/des agent(s) chélateur(s) ou tout mélange de ceux-ci.

5. Formulation injectable selon les revendications 1-4, dans laquelle le ou les antioxydant(s) de la formulation est/sont choisi (s) dans un groupe comprenant le monothioglycérol, le métabisulfite de sodium.

6. Formulation injectable selon les revendications 1-5, dans laquelle le(s) modificateur(s) de pH de la formulation est (sont) choisi(s) dans un groupe comprenant l'hydroxyde de sodium, l'acide chlorhydrique.

7. Formulation injectable selon les revendications 1-6, dans laquelle le(s) tampon(s) de la formulation est (sont) choisi(s) dans un groupe comprenant le phosphate de sodium dibasique, le phosphate de sodium monobasique.

8. Formulation injectable selon les revendications 1-7, dans laquelle dans la formulation, lorsqu'elle est diluée à l'aide d'eau pour injection ou de fluides IV aqueux conventionnels tels que du sérum physiologique, une solution de dextrose ou tout autre support parentéral, de telle sorte qu'une dose de 1 g de paracétamol soit administrée dans un volume de 20 ml de la formulation finale, aucun cristal n'apparaît pendant une période d'au moins 5 à 8 minutes.

9. Formulation injectable selon les revendications 1-8, dans laquelle les formulations peuvent être administrées par voie intramusculaire, par bolus intraveineux, par perfusion intraveineuse ainsi que par injection lente par bolus intraveineux.

10. Formulation injectable selon les revendications 1-9, dans laquelle l'injection lente par bolus intraveineux est stable de 2 à 10 minutes après dilution de la formulation.
